# EUROPEAN PATENT APPLICATION

(11) **EP 4 324 360 A1**
(43) Date of publication of application: **21.02.2024**
(21) Application number: 22787900.4
(22) Date of filing: 08.03.2022
(51) Int. Cl.: A47F 9/04, G07G 1/00, A61L 2/10

(54) **INACTIVATION METHOD AND PRECISE CALCULATION SYSTEM**

(30) Priority: 13.04.2021 JP 2021067671
(71) Applicant: Ushio Denki Kabushiki Kaisha, Tokyo 100-8150 (JP)
(72) Inventor: OHASHI, Hiroyuki, Tokyo 100-8150 (JP); SUZUKI, Shinji, Tokyo 100-8150 (JP); OWADA, Tatsushi, Tokyo 100-8150 (JP); ABE, Ryoji, Tokyo 100-8150 (JP)
(74) Representative: Maiwald GmbH
(86) International application number: PCT/JP2022/009886
(87) International publication number: WO 2022/219972

(57) **Abstract**

To appropriately decontaminate items related to checkout processing in a checkout area where the checkout processing is performed and prevent harmful microorganisms and/or viruses from infecting humans through the items, there is provided an inactivation method of emitting ultraviolet light in a space where a human is present to inactivate microorganisms and/or viruses existing in the space, comprising: a detection step for detecting an item related to checkout processing, the item being transferred to a checkout area where the checkout processing is performed for the item by a cashier at a checkout counter; an irradiation step for controlling, when the item is detected in the detection step, an ultraviolet light source that emits ultraviolet light in a wavelength range between 190 nm and 240 nm as ultraviolet light having a wavelength that inactivates the microorganisms and/or viruses and irradiating the checkout area with the ultraviolet light; and a removing step for transferring the item from the checkout area after the item has been irradiated with the ultraviolet light.

## Description

### TECHNICAL FIELD

The present invention relates to an inactivation method and a checkout system for inactivating harmful microorganisms and/or viruses.

### BACKGROUND ART

Medical facilities, schools, government offices, theaters, hotels, restaurants, and other facilities where people frequently gather, or enter and leave are environments in which microorganisms such as bacteria, mold, or the like tend to proliferate and also viruses tend to spread.

For example, harmful and highly infectious microorganisms and/or viruses are likely to proliferate on floors, walls, and other surfaces of a certain space in a facility when a person infected with the virus enters and leaves the space, or they are likely to float in the space. As a result, the virus may infect the next person who enters the space, and in some cases, the infectious disease may spread within the facility.

In order to resolve such adverse situation described above, measures to disinfect harmful microorganisms (e.g., infectious microorganisms) or inactivate viruses as described above are required in facilities where humans (or animals, as the case may be) gather, or enter and leave.

The surfaces surrounding the space, such as floors and walls, are decontaminated by workers by spraying disinfectant such as alcohol, wiping with a cloth soaked with disinfectant, or irradiating the surfaces with germicidal ultraviolet light. For microorganisms and/or viruses floating in the space, for example, sterilization and inactivation by ultraviolet light irradiation is performed.

Patent Literature 1 (Published Japanese Translation of PCT International Publication No. 2017-528258 A) discloses, as a decontamination apparatus for decontaminating an air-sealed room, an apparatus that irradiates the space to be decontaminated with ultraviolet light (i.e., UV-C light) when a user is not present to sterilize the space.

In the meantime, from the point of view of hygiene and other factors, some foods and beverages are sterilized in some cases by means of ultraviolet light.

Patent Literature 2 (Laid-open Publication of Japanese Patent Application No. 2007-110978 A) discloses an ultraviolet light sterilization apparatus that irradiates eggs loaded on a conveyor with ultraviolet light having the wavelength of 254 nm when the conveyor is in operation.

Patent Literature 3 (Laid-open Publication of Japanese Patent Application No. 2002-80017 A) discloses a sterilization apparatus that irradiates ultraviolet light from the underside of containers such as PET bottles being conveyed by a conveyor to sterilize the surface of the containers.

### LISTING OF REFERENCES

### PATENT LITERATURE

PATENT LITERATURE 1: Japanese Translation of PCT International Application Publication No. 2017-528258 A
PATENT LITERATURE 2: Laid-open Publication of Japanese Patent Application No. 2007-110978 A
PATENT LITERATURE 3: Laid-open Publication of Japanese Patent Application No. 2002-80017 A

### SUMMARY OF THE INVENTION

### PROBLEMS TO BE SOLVED BY THE INVENTION

Microorganisms and/or viruses harmful to human bodies are likely to adhere to surfaces of items that are contacted or approached by humans infected with the viruses, or the like.

For example, products displayed in convenience stores, supermarkets, department stores, or the like, are picked up by an unspecified number of customers in most cases. In other words, the above-mentioned harmful microorganisms and/or viruses are highly likely to adhere to the above-mentioned displayed products through an infected person, thus there is a risk of secondary infection of harmful microorganisms and/or viruses through the displayed products to customers who purchase the products and take them home. In addition, there is another risk of secondary infection of the above harmful microorganisms and/or viruses to a person in charge of checkout (e.g., cashier) who comes into contact with the products at a cash register counter or other place where the products are paid for.

Although the above-mentioned techniques disclosed in the Patent Literature 1 through 3 utilize ultraviolet light having the wavelength in the vicinity of 254 nm as germicidal ultraviolet light, such ultraviolet light having the wavelength in the vicinity of 254 nm are known to be light that adversely affects human bodies. For this reason, the above-mentioned techniques disclosed in the Patent Literature 1 through 3 are required to be limited to irradiating ultraviolet light in a space after humans have left or in a space where humans do not enter, in order to decontaminate the space. In other words, the above-mentioned techniques cannot be used in spaces where a human is present.

Therefore, the decontamination operation cannot be performed in an environment with a human in the vicinity, even when attempting to decontaminate products such as those mentioned above that are likely to come into contact with an unspecified number of people and may have harmful microorganisms and/or viruses adhered thereto.

The present invention has been made in order to solve the above-mentioned problems and an object thereof is to provide an inactivation method and a checkout system that are capable of appropriately decontaminating items related to checkout processing in a checkout area where the checkout processing is performed and inhibiting harmful microorganisms and/or viruses from infecting humans through the items.

### SOLUTION TO PROBLEMS

In order to solve the above mentioned problems, according to one aspect of the present invention, there is provided an inactivation method of emitting ultraviolet light in a space where a human is present to inactivate microorganisms and/or viruses existing in the space, comprising: a detection step for detecting an item related to checkout processing, the item being transferred to a checkout area where the checkout processing is performed for the item by a cashier at a checkout counter; an irradiation step for controlling, when the item is detected in the detection step, an ultraviolet light source that emits ultraviolet light in a wavelength range between 190 nm and 240 nm as ultraviolet light having a wavelength that inactivates the microorganisms and/or viruses and irradiating the checkout area with the ultraviolet light; and a removing step for transferring the item from the checkout area after the item has been irradiated with the ultraviolet light.

It makes it possible to irradiate the checkout area with ultraviolet light having a wavelength that inactivates microorganisms and/or viruses during the checkout processing for items by a cashier, so as to inactivate harmful microorganisms and/or viruses adhered to surfaces of the items related to the checkout processing (e.g., items to be processed for checkout). Therefore, it makes it possible to inhibit microorganisms and/or viruses from infecting humans through the items. In addition, since ultraviolet light in the wavelength range between 190 nm and 240 nm, which have little adverse effects on cells of humans and animals, are irradiated, it makes it possible to perform the inactivation processing by use of the ultraviolet light irradiation even in spaces where a human is present.

Furthermore, in the above inactivation method, the irradiation step may irradiate the checkout area with the ultraviolet light from a plurality of directions.

In this case, it makes it possible to irradiate items with the ultraviolet light from multiple sides, thereby performing the inactivation processing more appropriately.

Yet furthermore, in the above inactivation method, the detection step may detect that an item is placed in a processing area on the checkout counter, the processing area being an area included in the checkout area and in which the item is to be placed during the checkout processing; and the irradiation step may irradiate the processing area with the ultraviolet light when the detection step detects that the item is placed in the processing area.

In this case, it makes it possible to irradiate items in the process of checkout with the ultraviolet light. Thus, it makes it possible to carry the items in a decontaminated state out of the checkout area.

Yet furthermore, in the above inactivation method, the detection step may detect that an item is placed in a standby area on the checkout counter, the standby area being an area included in the checkout area and in which the item awaiting the checkout processing is to be placed; and the irradiation step may irradiate the standby area with the ultraviolet light when the detection step detects that the item is placed in the standby area.

In this case, it makes it possible to irradiate items awaiting the checkout processing with the ultraviolet light. Thus, it makes it possible to decontaminate the items before a cashier comes into contact with the items for the checkout processing.

Yet furthermore, in the above inactivation method, the item may be a to-be-purchased product and the checkout processing may be processing for checking out the to-be-purchased product.

In this case, it makes it possible to irradiate the checkout area where the checkout processing is performed with the ultraviolet light having the wavelength that inactivate microorganisms and/or viruses during the checkout processing of to-be-purchased items. Thus, it makes it possible to inhibit harmful microorganisms and/or viruses to infect a cashier or a purchaser of products through the products that may be in contact with an unspecified number of people and may have harmful microorganisms and/or viruses adhered thereto.

Yet furthermore, in the above inactivation method, the detection step may detect that an item is present in a reading area for reading product information assigned to the to-be-purchased product by a reader that reads the product information, the reading area being an area included in the checkout area; and the irradiation step may irradiate the reading area with the ultraviolet light when the detection step detects that the item is present in the reading area.

In this case, it makes it possible to irradiate individual products with the ultraviolet light more reliably.

Yet furthermore, in the above inactivation method, the detection step may detect that an item is present in a payment area for paying for the to-be-purchased product, the payment area being an area included in the checkout area; and the irradiation step may irradiate the payment area with the ultraviolet light when the detection step detects that the item is present in the payment area.

In this case, it makes it possible to irradiate cash, cards, and other items related to the checkout processing with the ultraviolet light. Thus, it makes it possible to suppress the secondary infection of harmful microorganisms and/or viruses through the items that are delivered between the cashier and the purchaser (e.g., customer) of the products.

Yet furthermore, in the above inactivation method, the irradiation step may stop irradiation of the ultraviolet light triggered by an event that the detection step no longer detects the item.

In this case, it makes it possible to prevent the checkout area from being unnecessarily exposed to the ultraviolet light. In addition, it makes it possible to prevent humans present in the checkout area from being exposed to the ultraviolet light, thereby minimizing the adverse effects of the irradiation of the ultraviolet light on such humans.

Yet furthermore, the above inactivation method may further comprise: a measurement step for measuring an irradiation amount of the ultraviolet light to the cashier, wherein the irradiation step may stop irradiation of the ultraviolet light when the irradiation amount to the cashier reaches an allowable upper limit of the irradiation amount to the cashier.

In this case, it makes it possible to perform the inactivation processing more appropriately while ensuring the safety of humans in the checkout area.

According to another aspect of the present invention, there is provided a checkout system comprising: a checkout counter; a checkout apparatus installed in the checkout counter; and an ultraviolet light irradiation apparatus. The ultraviolet light irradiation apparatus includes an ultraviolet light source configured to emit ultraviolet light in a wavelength range between 190 nm and 240 nm as ultraviolet light having a wavelength that inactivates the microorganisms and/or viruses, and a control unit configured to control irradiation of the ultraviolet light by the ultraviolet light source. The ultraviolet light irradiation apparatus further includes a detection unit configured to detect an item related to checkout processing, the item being transferred to a checkout area where the checkout processing is performed for a to-be-purchased product by a cashier at the checkout counter, the ultraviolet light source is configured to irradiate the checkout area with the ultraviolet light, and the control unit is configured to control, when the item is detected by the detection unit, the ultraviolet light source to irradiate the checkout area with the ultraviolet light to irradiate the item with the ultraviolet light.

It makes it possible to irradiate the checkout area with ultraviolet light having a wavelength that inactivates microorganisms and/or viruses during the checkout processing for to-be-purchased products by a cashier, so as to inactivate harmful microorganisms and/or viruses adhered to surfaces of the items related to the checkout processing (e.g., to-be-purchased products). Therefore, it makes it possible to inhibit microorganisms and/or viruses from infecting humans through the to-be-purchased products. In addition, since ultraviolet light in the wavelength range between 190 nm and 240 nm, which have little adverse effects on cells of humans and animals, are irradiated, it makes it possible to perform the inactivation processing by use of the ultraviolet light irradiation even in spaces where a human is present.

Furthermore, in the above checkout system, the ultraviolet light source may be configured to irradiate the checkout area with the ultraviolet light from at least one of above and below the checkout area.

In this case, it makes it possible to irradiate items with the ultraviolet light from multiple sides, thereby performing the inactivation processing more appropriately.

Yet furthermore, in the above checkout system, the ultraviolet light source may be configured to irradiate a processing area on the checkout counter with the ultraviolet light, the processing area being an area included in the checkout area and in which the to-be-purchased product is to be placed during the checkout processing, the detection unit may detect that an item is placed in the processing area on the checkout counter, and the control unit may control the ultraviolet light source to irradiate the processing area with the ultraviolet light when the detection unit detects that the item is placed in the processing area.

In this case, it makes it possible to irradiate to-be-purchased products in the process of checkout with the ultraviolet light. Thus, it makes it possible for a purchaser of products to take home the purchased product in a decontaminated state.

Yet furthermore, in the above checkout system, the ultraviolet light source may be configured to irradiate a standby area on the checkout counter with the ultraviolet light, the standby area being an area included in the checkout area and in which the to-be-purchased product awaiting the checkout processing is to be placed, the detection unit may detect that an item is placed in the standby area on the checkout counter, and the control unit may control the ultraviolet light source to irradiate the standby area with the ultraviolet light when the detection unit detects that the item is placed in the standby area.

In this case, it makes it possible to irradiate to-be-purchased products awaiting the checkout processing with the ultraviolet light. Thus, it makes it possible to decontaminate the to-be-purchased products before a cashier comes into contact with the to-be-purchased products for the checkout processing.

Yet furthermore, in the above checkout system, the ultraviolet light source may be arranged in a reader that reads product information assigned to the to-be-purchased product, the detection unit may detect that an item is present in a reading area for reading the product information by the reader, the reading area being an area included in the checkout area, and the control unit may control the ultraviolet light source to irradiate the reading area with the ultraviolet light when the detection unit detects that the item is present in the reading area.

In this case, it makes it possible to irradiate to-be-purchased products individually with the ultraviolet light more reliably.

Yet furthermore, in the above checkout system, the ultraviolet light source may be configured to irradiate a payment area for paying for the to-be-purchased product, the payment area being an area included in the checkout area, the detection unit may detect that an item is present in the payment area, and the control unit may control the ultraviolet light source to irradiate the payment area with the ultraviolet light when the detection unit detects that the item is present in the payment area.

In this case, it makes it possible to irradiate cash, cards, and other items for paying for to-be-purchased products with the ultraviolet light. Thus, it makes it possible to suppress the secondary infection of harmful microorganisms and/or viruses through the above items that are delivered between the cashier and the purchaser (e.g., customer) of products.

Yet furthermore, in the above checkout system, the checkout counter may be provided with a partition made of transparent material with an aperture for paying for the to-be-purchased product, the partition being arranged between a purchaser of a product and a cashier who performs the checkout processing and is different from the purchaser, the ultraviolet light source may be arranged at the aperture, and the detection unit may detect that the item is present in the payment area that is formed by the aperture.

In this case, it makes it possible to suppress the droplet infection between the cashier and the purchaser (e.g., customer) of products, and also suppress the secondary infection of harmful microorganisms and/or viruses through the items that are delivered between the cashier and the purchaser (e.g., customer) of the product.

Yet furthermore, in the above checkout system, the control unit may control the ultraviolet light source to stop irradiation of the ultraviolet light triggered by an event that the detection unit no longer detects the item.

In this case, it makes it possible to prevent the checkout area from being unnecessarily exposed to the ultraviolet light. In addition, it makes it possible to prevent humans present in the checkout area from being exposed to the ultraviolet light, thereby minimizing the adverse effects of the irradiation of the ultraviolet light on such humans.

Yet furthermore, the above checkout system may further comprise: a measurement unit configured to measure an irradiation amount of the ultraviolet light to the cashier, wherein the control unit may control the ultraviolet light source to stop irradiation of the ultraviolet light when the irradiation amount measured by the measurement unit reaches an allowable upper limit of the irradiation amount.

In this case, it makes it possible to perform the inactivation processing more appropriately while ensuring the safety of humans in the checkout area.

Yet furthermore, in the above checkout system, at least an area including and adjacent to an area in which the to-be-purchased product is to be placed on a surface of the checkout counter may constitute a reflective surface that reflects the ultraviolet light.

In this case, it makes it possible to reflect (e.g., diffusely reflect) the ultraviolet light irradiated onto the surface of the checkout counter, thereby irradiating the to-be-purchased products placed on the checkout counter with the ultraviolet light from multiple sides.

Yet furthermore, in the above checkout system, the checkout counter may be provided with a window that transmits the ultraviolet light on a surface of the checkout counter, at least in an area in which the to-be-purchased product is to be placed, and the ultraviolet light source may be arranged inside the checkout counter and below the window and is configured to emit the ultraviolet light in an upward direction.

In this case, it makes it possible to appropriately irradiate the ultraviolet light onto the contact surface of the to-be-purchased products, which are placed on the checkout counter, with the checkout counter.

Yet furthermore, in the above checkout system, the checkout counter may be provided with a product conveyance mechanism that loads and conveys the to-be-purchased product to a checkout position where the checkout processing is performed by the cashier, and the ultraviolet light source may be configured to irradiate the ultraviolet light onto an area upstream in a conveyance direction of the to-be-purchased product from the checkout position in a conveyance path of the to-be-purchased product by the product conveyance mechanism.

In this case, it makes it possible to irradiate to-be-purchased products with the ultraviolet light before the checkout processing. Thus, it makes it possible to decontaminate the to-be-purchased products before a cashier comes into contact with the to-be-purchased products for the checkout processing.

Yet furthermore, in the above checkout system, the product conveyance mechanism may be provided with a belt with a predetermined gap or rollers that rotate at a predetermined distance apart as a conveyance path on which the to-be-purchased product is to be placed, and the ultraviolet light source may be arranged below the conveyance path and emits the ultraviolet light in an upward direction.

In this case, it makes it possible to appropriately irradiate bottom surfaces of the to-be-purchased products with the ultraviolet light.

Yet furthermore, in the above checkout system, the conveyance path may be constituted with rollers that rotate at a predetermined distance apart, surfaces of the rollers may constitute reflective surfaces that reflect the ultraviolet light, and the ultraviolet light source may be configured to irradiate the rollers of which surfaces constitute the reflective surfaces with the ultraviolet light.

In this case, it makes it possible to reflect (e.g., diffusely reflect) the ultraviolet light irradiated onto the surfaces of the rollers, thereby irradiating the to-be-purchased products placed on the conveyance path with the ultraviolet light from multiple sides.

Yet furthermore, in the above checkout system, the checkout counter may be provided with a product passage member with an entrance/exit through which the to-be-purchased product conveyed by the product conveyance mechanism may pass, upstream in the conveyance direction of the to-be-purchased product from the checkout position in the conveyance path, and the ultraviolet light source may be arranged inside the product passage member.

In this case, it makes it possible to intensively irradiate the to-be-purchased products with the ultraviolet light before the checkout processing.

Yet furthermore, in the above checkout system, at least a part of an inner surface of the product passage member may constitute a reflective surface that reflects the ultraviolet light.

In this case, it makes it possible to reflect (e.g., diffusely reflect) the ultraviolet light in the inner surface of the product passage member, thereby irradiating the to-be-purchased products passing through the product passage member with the ultraviolet light from multiple sides.

### ADVANTAGEIOUS EFFECT OF THE INVENTION

According to the present invention, it makes it possible to appropriately decontaminate items related to checkout processing in a checkout area where the checkout processing is performed and inhibit harmful microorganisms and/or viruses from infecting humans through the items.

The above mentioned and other not explicitly mentioned objects, aspects and advantages of the present invention will become apparent to those skilled in the art from the following embodiments (detailed description) of the invention by referring to the accompanying drawings and the appended claims.

### BRIEF DESCRIPTION OF DRAWINGS

FIG. 1 is a schematic diagram illustrating an exemplary configuration of a checkout system according to the present embodiment.
FIG. 2 is a schematic diagram illustrating another exemplary configuration of the checkout system.
FIG. 3 is a schematic diagram illustrating yet another exemplary configuration of the checkout system.
FIG. 4 is a schematic diagram illustrating yet another exemplary configuration of the checkout system.
FIG. 5 is a schematic diagram illustrating an exemplary configuration of an ultraviolet light irradiation unit.
FIGs. 6A and 6B are schematic diagrams illustrating an exemplary configuration of an excimer lamp.
FIGs. 7A and 7B are schematic diagrams illustrating another exemplary configuration of the excimer lamp.
FIGs. 8A and 8B are schematic diagrams illustrating yet another exemplary configuration of the excimer lamp.
FIG. 9 is a chart exemplarily illustrating the ultraviolet light absorption spectrum of the protein.
FIG. 10 is a schematic diagram illustrating another exemplary configuration of the ultraviolet light irradiation unit.

### DESCRIPTION OF EMBODIMENTS

Hereinafter, non-limiting embodiments of the present invention will be described in detail with reference to the accompanying drawings.

The present embodiment will exemplarily describe a checkout system with a checkout counter for performing checkout processing of to-be-purchased products as a checkout counter that handles the checkout of items. Here, the checkout counter may be a cash register counter that checks out products displayed in, for example, convenience stores, supermarkets, department stores, or the like.

The checkout system according to the present embodiment is equipped with an ultraviolet light irradiation apparatus that performs ultraviolet light irradiation in a checkout area where the checkout processing of products by a cashier at the checkout counter (e.g., cash register counter) is performed. In other words, the ultraviolet light (hereinafter referred to as "UV light" or simply referred to as "UV") irradiation apparatus performs the UV irradiation in a space where a human is present. The UV irradiation apparatus according to the present embodiment irradiates UV light having a wavelength between 190 nm and 240 nm, which have little adverse effects on cells of humans and animals, into the space, and inactivates harmful microorganisms and/or viruses existing on a surface of objects or in various spaces in the space concerned.

The term "inactivation" according to the present embodiment refers to the death of microorganisms and/or viruses (or the loss of infectivity and/or toxicity thereof).

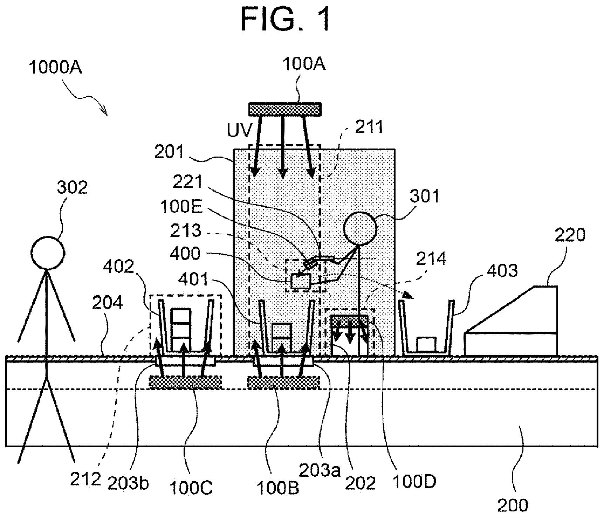

FIG. 1 is a schematic diagram illustrating an exemplary configuration of a checkout system 1000A according to the present embodiment.

The checkout system 1000A includes multiple ultraviolet light irradiators (i.e., UV irradiators) 100A to 100E, which constitute the UV irradiation apparatus, a cash register counter (i.e., checkout counter) 200, and a cash register (i.e., checkout apparatus) 220.

The cash register counter 200 is a platform on which shopping baskets containing products 400 are placed. The cash register counter 200 is a face-to-face counter, with a person in charge of checkout 301, serving as a cashier, standing on one side across the cash register counter 200 and a purchaser of a product (e.g., customer) 302 standing on the other side.

It should be noted that, although in some cases products 400 not added to a shopping basket may be placed directly on the cash register counter 200, the present embodiment will describe a certain case in which a customer 302 adds products 400 to a shopping basket and places the shopping basket on the cash register counter 200, and a cashier 301 takes the products 400 out of the shopping basket placed on the register counter 200 and performs the checkout processing for the products 400.

More specifically, the customer 302 selects a product 400 to be purchased from a display shelf or the like, adds the product 400 to the shopping basket, transports the shopping basket to a checkout area where the cash register counter 200 is installed, and places the shopping basket on the cash register counter 200. On the cash register counter 200, a processing area 211 is predefined where the shopping basket (i.e., shopping basket in process) 401 containing products 400, which are being checkout-processed by the cashier 301, is placed. The shopping basket placed on the cash register counter 200 by the customer 302 is transferred to the processing area 211 by the cashier 301 or the customer 302 him/herself. Subsequently, the cashier 301 performs the checkout processing for the products 400 in the shopping basket 401 placed in the processing area 211. Furthermore, upstream of the processing area 211 on the cash register counter 200 (left side of FIG. 1), a standby area 212 is predefined where the shopping basket 402 containing products 400 waiting to be checked out (i.e., checkout-standby shopping basket) is placed. While the shopping basket in process 401 is being checked out by the cashier 301, a next customer 302 places the shopping basket in the standby area 212.

The cashier 301 takes unpaid products 400 one by one from the shopping basket in process 401, reads product information (e.g., bar code, QR code (registered trademark), or the like) assigned to the product 400 by a reader 221 connected wirelessly or wired to the cash register 220, and adds the products 400 to a checked-out shopping basket 403. Here, the checked-out shopping basket 403 is placed on the cash register counter 200, for example, downstream of the processing area 211 (right side of FIG. 1).

The cash register 220 calculates the total purchase amount based on the information read by the reader 221 and presents the calculated purchase amount to the customer 302. The customer 302 pays the cashier 301 based on the purchase amount presented, and the checkout is completed. After the checkout is completed, the checked-out shopping basket 403 is handed to the customer 302, and the customer 302 removes the products 400 from the checkout area and takes the products 400 to go.

The reader 221 may be, for example, a hand-held type bar code reader. The reader 221 includes a reading unit (not shown) that optically reads the product information, reads the product information of products 400 in a reading area 213 adjacent to the reading unit, and transmits the read information to the cash register 220.

Although FIG. 1 illustrates a certain case in which the reader 221 is the hand-held type barcode reader, alternatively, the reader 221 may be, for example, a fixed barcode reader.

Furthermore, the cash register counter 200 may be equipped with a transparent partition 201 that is arranged between the cashier 301 and the customer 302 facing each other across the cash register counter 200. The partition 201 is designed to prevent droplets, which may contain viruses or other substances scattered from one of the cashier 301 and the customer 302 facing each other, from reaching the other.

An aperture 202 may be provided at the bottom of the partition 201. The aperture 202 may be an opening for payment. In this case, the area formed by the aperture 202 constitutes a payment area 214, and the customer 302 pays for the product in the payment area 214.

A transparent curtain may be installed in place of the transparent partition 201.

The UV irradiators 100A to 100E are equipped with UV light sources that emit UV light in the wavelength range between 190 nm and 240 nm, respectively, and emit the above UV light in a predefined area included in the checkout area to inactivate microorganisms and/or viruses adhered to the surface of items in the irradiated area, or microorganisms and/or viruses floating in the spaces in the irradiated area.

The irradiation of UV light by the UV light sources equipped in the UV irradiators 100A to 100E is controlled by the control unit. Here, the above control unit may be provided in each of the UV irradiators 100A to 100E, or alternatively, a common control unit may be provided separately from the UV irradiators 100A to 100E.

The UV irradiator 100A is arranged above the processing area 211 (e.g., on the ceiling) and emits UV light in a downward direction. The UV light emitted from the UV irradiator 100A is irradiated onto the shopping basket in process 401, which is placed in the processing area 211 on the cash register counter 200, the products 400 added to the shopping basket in process 401, and other products 400 existing in the processing area 211 (e.g., products 400 being grasped by the cashier 301 for the checkout processing).

The UV irradiator 100B is arranged below the processing area 211 and emits UV light in an upward direction.
A window member that transmits UV light (i.e., UV transmissive window) 203a is provided at the position where the shopping basket in process 401 is placed on the surface of the cash register counter 200. The UV irradiator 100B is arranged inside the cash register counter 200 and below the UV transmissive window 203a.

The UV light emitted from the UV irradiator 100B transmits the UV transmissive window 203a and is irradiated onto the shopping basket in process 401, which is placed in the processing area 211 on the cash register counter 200, the products 400 added to the shopping basket in process 401, and other products 400 existing in the processing area 211 (e.g., products 400 being grasped by the cashier 301 for the checkout processing).

The UV irradiator 100C is arranged below the standby area 212 and emits UV light in the upward direction.
A window member that transmits UV light (i.e., UV transmissive window) 203b is provided at the position where the checkout-standby shopping basket 401 is placed on the surface of the cash register counter 200. The UV irradiator 100C is arranged inside the cash register counter 200 and below the UV transmissive window 203b.

The UV light emitted from the UV irradiator 100C transmits the UV transmissive window 203b and is irradiated onto the checkout-standby shopping basket 402, which is placed in the standby area 212 on the cash register counter 200, and the products 400 added to the checkout-standby shopping basket 402.

Another UV irradiator that emits UV light in the downward direction, similarly to the UV irradiator 100A, may be provided above the standby area 212. In this way, the UV irradiators are arranged above and below the area in which the shopping basket is placed on the cash register counter 200, respectively. Thus, it makes it possible to irradiate the products 400 with the UV light from multiple directions.

Furthermore, the UV irradiators 100B and 100C are arranged inside the cash register counter 200 and emit UV light through the UV transmissive windows 203a and 203b, respectively. Thus, it makes it possible to appropriately irradiate the shopping baskets with UV light from below the shopping baskets placed on the cash register counter 200. This enables the inactivation processing to be appropriately performed on the surface of the shopping basket in contact with the cash register counter 200 as well.

The UV irradiator 100D is arranged at the top of the aperture 202 for payment, which is situated at the bottom of the transparent partition 201 positioned between the cashier 301 and the customer 302, and emits UV light in the downward direction. The UV light emitted from the UV irradiator 100D is irradiated onto items for payment, such as cash, cards, smart phones, gift certificates, and other cashless payment tools placed in the payment area 214 formed by the aperture 202.

The UV irradiator 100E is arranged in the vicinity of the reading unit for reading the product information in the reader 221. The UV light emitted from the UV irradiator 100E is irradiated onto the product 400 facing the above reading unit when the product information is read by the reader 221. It makes it possible to individually inactivate the surface of each of the products 400 to be processed for checkout more reliably.

Each of the UV irradiators 100A to 100E is equipped with a sensor (i.e., detection unit), the illustration of which is omitted, and the control unit described above controls the UV light source to emit UV light at the timing when an item is detected by the sensor described above.

More specifically, the UV irradiators 100A and 100B irradiate the processing area 211 with the UV light at the timing when the sensors detect that the shopping basket in process 401 is placed in the processing area 211 on the cash register 200, respectively, thereby irradiating items in the processing area 211 with the UVlight. Subsequently, the UV irradiators 100A and 100B stop irradiating the UV light at the timing when the sensors detect that the shopping basket in process 401 is transferred (removed) from the processing area 211 on the cash register counter 200.

Similarly, the UV irradiator 100C irradiates items in the standby area 212 with the UV light at the timing when the sensor detects that the checkout-standby shopping basket 402 is placed in the standby area 212 on the cash register counter 200. Subsequently, the UV irradiator 100C stops irradiating the UV light at the timing when the sensor detects that the checkout-standby shopping basket 402 is transferred (removed) from the standby area 212 on the cash register counter 200.

The UV irradiator 100D irradiates items existing in the payment area 214 with the UV light at the timing when the sensor detects that an item (such as money for payment) is placed in the payment area 214, and stops irradiating the UV light at the timing when the sensor detects that the item is transferred (removed) from the payment area 214. Likewise, the UV irradiator 100E irradiates an item (e.g., product) existing in the reading area 213 with the UV light at the timing when the sensor detects the item (e.g., product) facing the reading unit in the reading area 213 of the reader 221, and stops irradiating the UV light at the timing when the sensor detects that the item is transferred (removed) from the reading area 213.

In this way, it makes it possible to irradiate an appropriate area with the UV light at the appropriate time, thereby performing the inactivation processing more effectively.

Although the above describes a certain case in which the UV irradiation is stopped at the timing when the sensor no longer detects an item, the timing for stopping UV irradiation is not limited to the above. It may be sufficient to stop the UV irradiation triggered by an event that the sensor no longer detects an item. For example, the UV irradiation may be stopped after a predetermined time (e.g., several seconds to several tens of seconds) after the sensor no longer detects an item.

Furthermore, although the above describes a certain case in which the UV irradiators 100A to 100E are equipped with sensors, respectively, alternatively, UV irradiation by multiple UV irradiators may be controlled based on a detection signal from a single sensor.
For example, the UV light may be irradiated from the UV irradiators 100A to 100E, respectively, at the timing when the sensor installed in the UV irradiator 100A detects that the shopping basket in process 401 is placed on the processing area 211 on the register counter 200.

Yet furthermore, although the above describes a certain case in which the UV irradiation is stopped triggered by an event that the sensor no longer detects an item, alternatively, the UV irradiation apparatus may be equipped with a measurement unit that measures the irradiation amount (i.e., irradiation dose) of UV light to the cashier 301, and may continuously or intermittently perform the UV irradiation until the irradiation amount reaches the allowable upper limit. Here, the above allowable upper limit may be the allowable limit value (TLV: Threshold Limit Value) for the irradiation amount of UV light to the human body per day (i.e., 8 hours), which is specified for each wavelength according to the ACGIH (American Conference of Governmental Industrial Hygienists) and JIS Z 8812 (Measurement method of harmful ultraviolet radiation). Otherwise, the above allowable upper limit may be a value lower than the TLV, which is preset based on the above TLV.

In this case, for example, the irradiation amount of UV light from the time when the cashier 301 stands at the cash register counter 200 is accumulated, and when the accumulated value reaches the preset allowable upper limit, the UV irradiation may be stopped. The above measurement unit may be configured to measure and accumulate the irradiation amount of UV light to the cashier 301 by a UV sensor carried by the cashier 301 or installed in the vicinity of the cashier 301. Alternatively, the UV irradiation apparatus may be configured to preset the irradiation amount of UV light per unit time to the cashier 301, and estimate the accumulated value of the irradiation amount of UV light to the cashier 301 using timer counting.

As shown in FIG. 1, the surface of the cash register counter 200 may be a reflective surface 204 that reflects the UV light having the wavelength between 190 nm and 240 nm (preferably, 222 nm). The reflective surface 204 is made of a reflective material that reflects the above UV light. The reflective surface 204 may be provided on the surface of the cash register counter 200, at least in an area thereon including and adjacent to the area in which the shopping baskets (i.e., shopping basket in process 401 and checkout-standby shopping basket 402) containing unpaid products 400 are to be placed (i.e., processing area 211 and standby area 212). In this way, the UV light irradiated from above the cash register counter 200 onto the surface of the cash register counter 200 is reflected (e.g., diffusely reflected) by the reflective surface 204 and irradiated onto the unpaid products 400.

As described above, the checkout system 1000A according to the present embodiment is capable of irradiating the checkout area where the checkout processing is performed with UV light during the checkout processing for the products 400 by the cashier 301 at the cash register counter 200. Therefore, it makes it possible to irradiate items related to the checkout processing with the UV light and appropriately inactivate microorganisms and/or viruses adhered to the surface of the items. Here, the items related to the checkout processing include unpaid products 400, shopping baskets containing the unpaid products 400, the payment tool for the products 400 (e.g., cash, cards, and the like) existing in the checkout area.
This allows the cashier 301 to perform the checkout processing for the products 400 to which the inactivation processing has been performed. Also, the customer 302 may take home the products 400 to which the inactivation processing has been performed. Furthermore, the cashier 301 and the customer 302 may deliver the items (cash, or the like) to which the inactivation processing has been performed.

As a result, it makes it possible to suppress the secondary infection of harmful microorganisms and/or viruses through the items that may have the above microorganisms and/or viruses adhered thereto due to contact with an unspecified number of people.

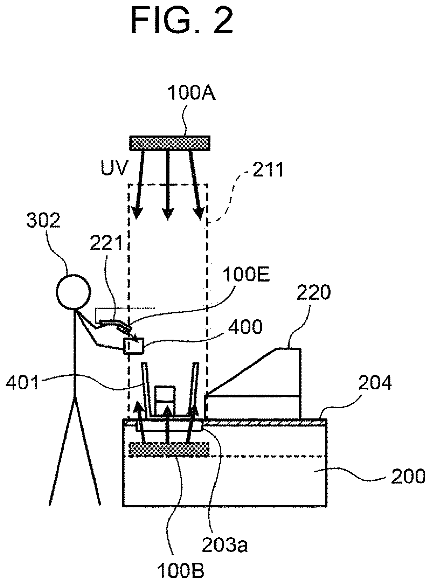

Although the above embodiment describes a certain case in which the cash register counter 200 is a face-to-face counter, alternatively, as exemplarily shown in FIG. 2, the present embodiment may be applied to a self-checkout system (self-checkout register) where customers 302 themselves behave as cashiers to perform the checkout processing. In FIG. 2, components having the similar configuration to the checkout system 1000A shown in FIG. 1 are denoted with the same reference signs as in FIG. 1.

Furthermore, although the above embodiment describes a certain case in which the reader 221 is a bar code reader, the method of reading the product information is not limited to the above. For example, the product information may be read using the near field wireless communication such as RFIDs. In this case, a shopping basket containing unpaid products tagged with RFIDs is placed in a predetermined area where an RFID tag reader is installed at the cash register counter, for example, and data of respective RFID tags of the products is collectively read by the RFID tag reader. As a result, in this case as well, by irradiating the area in which the above shopping basket is placed with UV light, it makes it possible to appropriately perform the inactivation processing to each of the products.

Yet furthermore, the cash register counter 200 may be equipped with a product conveyance mechanism that conveys the shopping basket containing the products 400 or the products 400 themselves to the checkout position where the checkout processing is performed by the cashier (i.e., position where the shopping basket in process 401 is placed in the processing area 211).

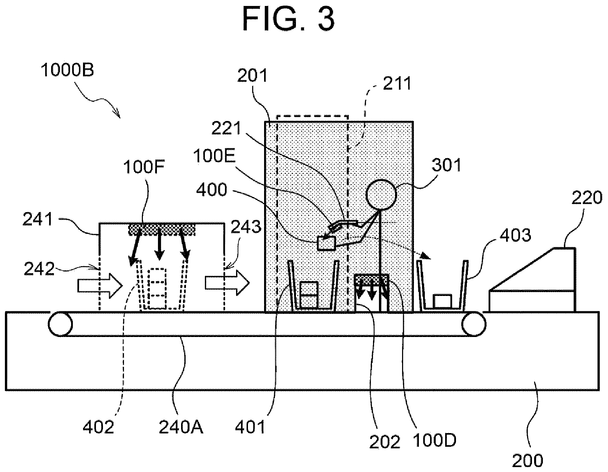

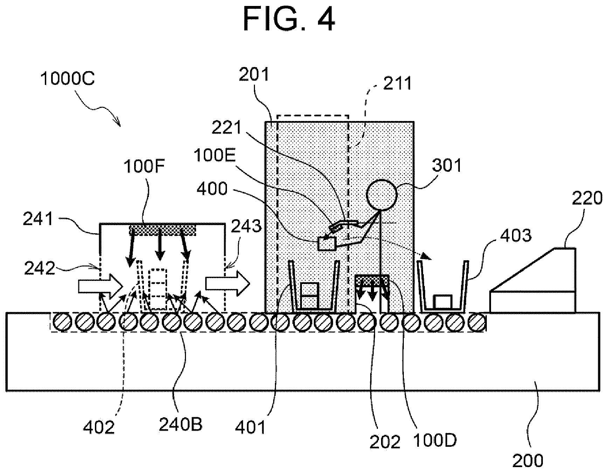

FIG. 3 is a schematic diagram illustrating an exemplary configuration of a checkout system 1000B in which the cash register counter 200 is equipped with a conveyor belt type conveyance mechanism 240A as the product conveyance mechanism, and FIG. 4 is a schematic diagram illustrating an exemplary configuration of a checkout system 1000C in which the cash register counter 200 is equipped with a roller type conveyance mechanism 240B as the product conveyance mechanism. In FIGs. 3 and 4, components having a similar configuration to the checkout system 1000A shown in FIG. 1 are denoted with the same reference signs as in FIG. 1.

Although not specifically illustrated, similarly to the checkout system 1000A shown in FIG. 1, the checkout systems 1000B and 1000C may be equipped with the UV irradiators 100A to 100C, respectively. Here, the UV irradiators (i.e., UV irradiators corresponding to the UV irradiators 100B and 100C in FIG. 1), which irradiate the products placed on the product conveyance mechanisms 240A and 240B with the UV light from below the placed products, are positioned below the conveyance paths of the product conveyance mechanisms 240A and 240B and emit the UV light in the upward direction.

The timing of UV irradiation by respective UV irradiators may be the same as in the checkout system 1000A described above. For example, it is conceivable to define an area corresponding to the processing area 211 or the standby area 212 shown in FIG. 1 on the conveyance paths of the product conveyance mechanisms 240A and 240B, and the UV light is irradiated when a sensor detects that the product 400 on the conveyance path has reached the above area.

The conveyor belt type conveyance mechanism 240A is equipped with a conveyor belt as the conveyance path for placing and conveying the products 400. The conveyor belt is a belt with a meshed structure and has predetermined gaps. The UV light, which is emitted upward from the UV irradiator located underneath the conveyance path, passes through the above mesh and is irradiated onto the products 400 on the conveyance path.

The roller type conveyance mechanism 240B is equipped with rollers that rotate at a predetermined distance apart as the conveyance path for placing and conveying the products 400. The UV light, which is emitted upward from the UV irradiator located underneath the conveyance path, passes through the gap between the rollers and is irradiated onto the products 400 on the conveyance path.

Furthermore, in the checkout systems 1000B and 1000C according to the present embodiment, a part of the conveyance path of the product conveyance mechanisms 240A and 240B may be provided with a product passage member 241 through which the product 400 conveyed on the conveyance path may pass. The product passage member 241 is provided upstream in the product conveyance direction (left side in FIGs. 3 and 4) of the products 400 from the checkout position in the conveyance path of the products 400.

The product passage member 241 has a box-like casing structure, for example, and encloses at least a part of the conveyance path of the product conveyance mechanisms 240A and 240B. The product passage member 241 includes an entrance 242 and an exit 243 through which the product 400 passes on the side faces opposite to each other in the conveyance direction of the product 400 by the product conveyance mechanism. In other words, the product passage section 241 functions as a tunnel through which the products 400 passes.

A UV irradiator 100F that emits UV light in the downward direction may be provided on the upper surface in the interior of the product passage member 241. In this case, the UV irradiator 100F irradiates the products 400 passing through the interior of the product passage member 241 with the UV light in the wavelength range between 190 nm and 240 nm to inactivate viruses and/or microorganisms adhered to the surface of the products 400.

The inner surface of the product passage member 241 may constitute a reflective surface that reflects the UV light emitted from the UV irradiator 100F. For example, the box-like casing structure constituting the product passage member 241 itself may be made of a reflective member that reflects UV light, or alternatively, the inner surface of the product passage member 241 may be given a UV light reflection function. By constituting the inner surface of the product passage member 241 with the reflective surface as described above, reflection (e.g., diffuse reflection) of UV light occurs on the inner surface of the product passage member 241. Thus, it makes it possible to irradiate the products 400 passing through the product passage member 241 with UV light from multiple sides.

It should be noted that, since the entrance 242 and the exit 243 of the product passage member 241 are kept open, the UV light emitted from the UV irradiator 100F and reflected on the inner surface of the product passage member 241 is likely to leak out of the product passage member 241 through the entrance 242 and the exit 243. However, as the UV light emitted from the UV irradiator 100F is light having the wavelength range that has little adverse effects on the human bodies, even when the UV light leaking from the entrance 242 and the exit 243 of the product passage member 241 are irradiated to humans (e.g., the cashier 301) positioned in the vicinity of the product passage member 241, it will hardly cause damage to such humans.

In the case of the roller type conveyance mechanism 240B shown in FIG. 4, the surfaces of the rollers may be reflective surfaces that reflect UV light, respectively. For example, a reflective member that reflects UV light may be provided on the roller surface, or the roller itself may be made of a reflective member that reflects UV light. For example, by constituting the surfaces of the rollers arranged inside the product passage member 241 with reflective surfaces as described above, when UV irradiation by the UV irradiator 100F is performed inside the product passage member 241, reflection (e.g., diffuse reflection) of UV light occurs on the surfaces of the rollers. Thus, it makes it possible to irradiate the products 400 passing inside the product passage member 241 from multiple sides.

When the products 400 are directly placed on the rollers without being added to a shopping basket, the products 400 move as the rollers rotate, so that the UV light emitted from above and below the conveyance path (i.e., rollers) irradiates the products 400 almost uniformly.

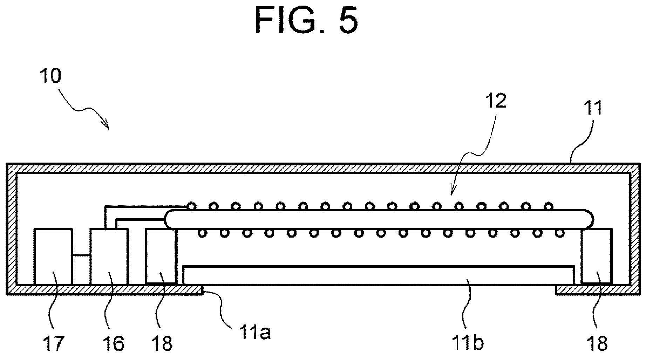

FIG. 5 is a schematic diagram illustrating an exemplary configuration of the UV irradiation unit 10 arranged in each of the UV irradiators 100A to 100F. Although a certain case will be described here in which the UV irradiators 100A to 100F have the same configuration and each of the UV irradiators is equipped with a control unit that controls irradiation, the configuration of each of the UV irradiators is not limited to the above.

The UV irradiation unit 10 includes a housing 11 made of conductive metal and a UV light source 12 housed inside the housing 11.

The UV light source 12 may be, for example, a KrCI excimer lamp that emits UV light having a center wavelength of 222 nm. It should be noted that the UV light source 12 is not limited to KrCI excimer lamps, but may be any light source that emits UV light in the wavelength range between 190 nm and 240 nm.

The UV irradiation unit 10 includes a power supply unit 16 that supplies power to the excimer lamp 12 and a control unit 17 that controls the irradiation and non-irradiation by the excimer lamp 12 and the light intensity of the UV light emitted from the excimer lamp 12. The excimer lamp 12 is supported by the support member 18 in the housing 11.

The housing 11 has an aperture 11a that serves as a light emitting window. The aperture 11a is equipped with a window member 11b. The window member 11b may include, for example, a UV transmissive member made of quartz glass or an optical filter that blocks unwanted light.

It should be noted that multiple excimer lamps 12 may be arranged in the housing 11, and the number of excimer lamps 12 is not particularly limited.

As the optical filter describe above, for example, a wavelength selective filter that transmits light in the wavelength range between 200 nm and 237 nm and cuts light in the other UV-C wavelength range (i.e., 200 nm to 280 nm) may be used.

Here, for example, a dielectric multilayer filter with HfO₂ and SiO₂ layers may be used as the wavelength selective filter.

As such, by providing an optical filter on the light emitting window, even when a small amount of light harmful to human bodies is emitted from the excimer lamp 12, it makes it possible to prevent the light from leaking to the outside of the housing 11 more reliably.

Alternatively, an optical filter having a dielectric multilayer film with SiO₂ and Al₂O₃ layers may be used as the wavelength selective filter.

However, when the optical filter having the dielectric multilayer film with HfO₂ and SiO₂ layers is used as the wavelength selective filter, the total number of layers may be reduced as compared to the optical filter having the dielectric multilayer film with SiO₂ and Al₂O₃ layers. Therefore, in this case, it makes it possible to increase the transmittance of UV light at an incident angle of 0°.

Hereinafter, an exemplary configuration of the excimer lamp 12 used as the UV light source in the UV irradiation unit 10 will be described more specifically.

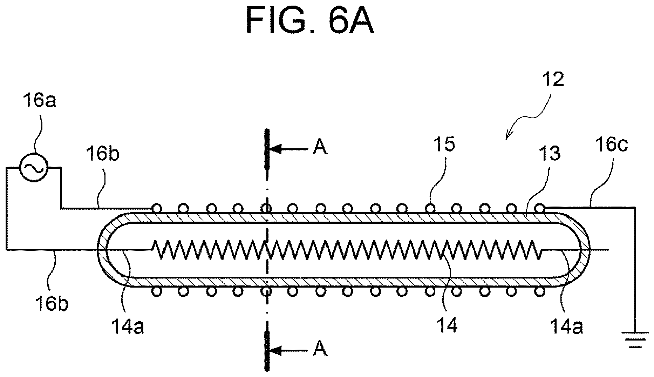

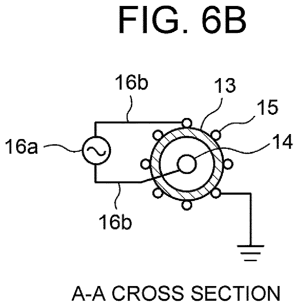

FIG. 6A is a schematic diagram illustrating an exemplary cross section of the excimer lamp 12 in the direction of the tube axis, and FIG. 6B is a schematic diagram illustrating the A-A cross section of FIG. 6A.

As shown in FIGs. 6A and 6B, the excimer lamp 12 is equipped with a discharge vessel 13, which is a longitudinal straight circular tube airtightly sealed at both ends. The discharge vessel 13 is made of a light transmissive dielectric material that transmits UV light, such as synthetic quartz glass or fused quartz glass. A discharge space is formed inside the discharge vessel 13, and the discharge space is filled with a rare gas and a halogen gas as the barrier discharge gas (hereinafter also referred to as the "discharge gas") that generates UV light. According to the present embodiment, krypton (Kr) is used as the rare gas and chlorine gas (Cl₂) is used as the halogen gas.

Alternatiely, a mixture of krypton (Kr) and bromine (Br₂) may be used as the discharge gas. In this case, the excimer lamp (i.e., KrBr excimer lamp) emits UV light having a center wavelength of 207 nm.

A first electrode (i.e., inner electrode) 14 is arranged in the discharge space inside the discharge vessel 13. The inner electrode 14 is a coiled electrode formed by coiling a metal strand made of an electrically conductive and heat resistant metal, such as tungsten, into a coil with a coil diameter smaller than the inner diameter of the discharge vessel 13. The inner electrode 14 extends along the central axis (i.e., tube axis) of the discharge vessel 13 and is arranged such that the inner electrode 14 does not contact the inner surface of the discharge vessel 13.
One ends of lead members 14a for the inner electrode 14 are electrically connected to both ends of the inner electrode 14, respectively. The other ends of the lead members 14a for the inner electrode 14 protrude outwardly from the outer end surfaces of the discharge vessel 13, respectively.

A second electrode (i.e., outer electrode) 15 is arranged on the outer circumference of the discharge vessel 13. The outer electrode 15 is a mesh type electrode of metal strands made of an electrically conductive and heat resistant metal, such as tungsten. The outer electrode 15 is provided to extend along the outer circumference of the discharge vessel 13 in the direction of the central axis of the discharge vessel 13. Referring to the excimer lamp 12 shown in FIGs. 6A and 6B, the outer electrode 15, which is a mesh type electrode, has a cylindrical outer shape and is arranged in close proximity to the outer circumference of the discharge vessel 13.

With the above configuration, a discharge region is formed in the discharge space in the area where the inner electrode 14 and the outer electrode 15 face each other through the tube wall (e.g., dielectric material wall) of the discharge vessel 13.

Furthermore, one end of the outer electrode 15 and the other end of one of the lead members 14a for the inner electrode 14 are each connected to a high frequency power supply 16a provided in the power supply unit 16 (as shown in FIG. 5) via a power feeder wire 16b. The high frequency power supply 16a is a power supply capable of applying a high frequency voltage between the inner electrode 14 and the outer electrode 15.

The other end of the outer electrode 15 is electrically connected to one end of a lead wire 16c, and the other end of the lead wire 16c is grounded. In other words, the outer electrode 15 is grounded via the lead wire 16c. Referring to the excimer lamp 12 shown in FIGs. 6A and 6B, one of the lead members 14a for the inner electrode 14 is integral with the power feeder wire 16b.

When high frequency power is applied between the inner electrode 14 and outer electrode 15, a dielectric barrier discharge occurs in the discharge space. The dielectric barrier discharge excites the atoms of the discharge gas (i.e., barrier discharge gas) sealed in the discharge space so as to produce excited dimers (i.e., exciplex). When the excited dimers return to the original state (i.e., ground state), a unique luminescence (i.e., excimer luminescence) is produced. In other words, the above discharge gas is an excimer luminescence gas.

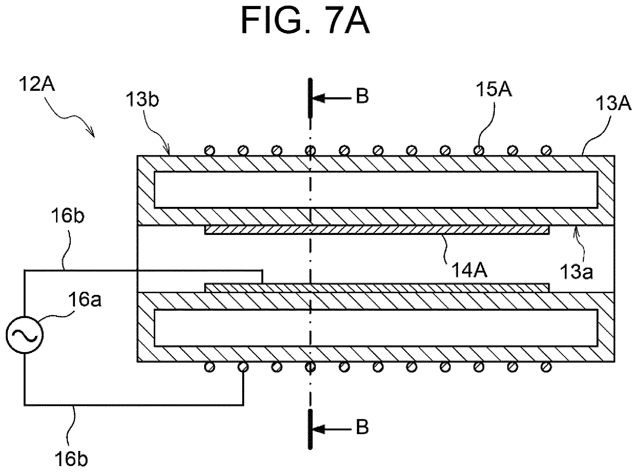

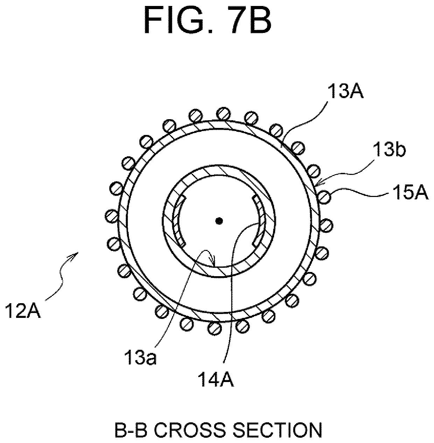

It should be noted that the configuration of excimer lamps is not limited to that shown in FIGs. 6A and 6B. For example, the excimer lamp may be configured with a double-tube structure discharge vessel 13A, as an excimer lamp 12A shown in FIGs. 7A and 7B.

The discharge vessel 13A equipped with the excimer lamp 12A includes a cylindrical outer tube and a cylindrical inner tube that is coaxially arranged inside the outer tube and has a smaller inner diameter than the outer tube. The outer tube and the inner tube are sealed at both ends, in the left to right direction in FIG. 7A, and an annular-shaped inner space is formed between the outer tube and the inner tube. The inner space is filled with the discharge gas.

A membrane-shaped first electrode (i.e., inner electrode) 14A is provided on the inner wall surface 13a of the inner tube, and a mesh or net-shaped second electrode (i.e., outer electrode) 15A is provided on the outer wall surface 13b of the outer tube.
The inner electrode 14A and outer electrode 15A are electrically connected to the high frequency power supply 16a via the power feeding wire 16b, respectively.

A high frequency AC voltage is applied between the inner electrode 14A and outer electrode 15A by the high frequency power supply 16a, which applies voltage to the discharge gas through tube bodies of the outer and inner tubes, so as to cause a dielectric barrier discharge in the discharge space in which the discharge gas is enclosed. This excites the atoms of the discharge gas to form excited dimers, and excimer luminescence is produced when those atoms transition to the ground state.

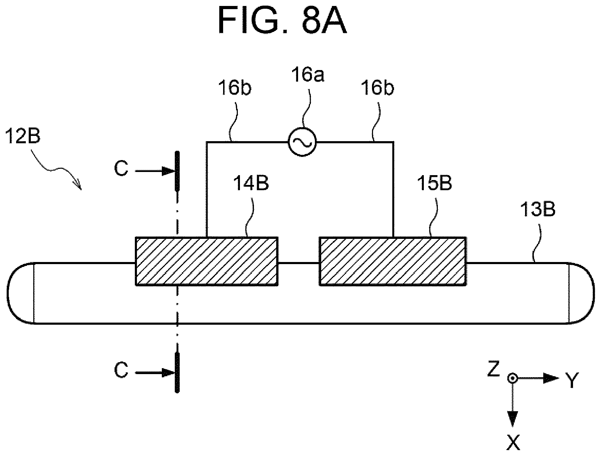

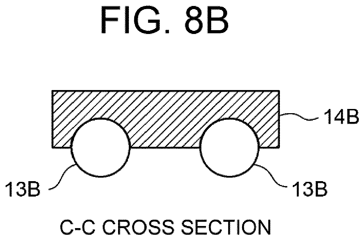

The excimer lamp may be configured, for example, with a pair of electrodes (i.e., first electrode 14B and second electrode 15B) arranged on one side of the discharge vessel 13B, as in an excimer lamp 12B shown in FIGs. 8A and 8B. Here, as an example, two discharge vessels 13B are arranged side by side in the Z direction in FIG. 8A.

As shown in FIG. 8A, the first electrode 14B and the second electrode 15B are arranged on the side opposite the light extraction surface in the discharge vessel 13B (i.e., the side in the -X direction), spaced from each other in the tube axis direction (i.e., Y direction) of the discharge vessel 13B.

The discharge vessel 13B is arranged to straddle those two electrodes 14B and 15B while contacting the two electrodes 14B and 15B. More specifically, each of the two electrodes 14B and 15B has a concave groove extending in the Y direction, and the discharge vessel 13B is fitted into the concave grooves of the electrodes 14B and 15B.

The first electrode 14B and the second electrode 15B are electrically connected to the high frequency power supply 16a via the power feeding wire 16b, respectively. When a high frequency AC voltage is applied between the first electrode 14B and the second electrode 15B, an excited dimers are generated in the inner space of the discharge vessel 13B, and excimer light is emitted from the light extraction surface (i.e., in the +X direction) of the excimer lamp 12B.

Here, the first and second electrodes 14B and 15B may be made of a metal material that is reflective to the light emitted from the excimer lamp 12B. In this case, the light emitted from the discharge vessel 13B in the -X direction can be reflected and travel in the +X direction. The first and second electrodes 14B and 15B may be made of aluminum (Al) or stainless steel, for example.

It should be noted that, as described above, excimer lamps may generate high frequency noise because high frequency power is applied to the excimer lamps to perform high frequency lighting. However, according to the present embodiment, as described above, by configuring the housing 11 that houses the excimer lamp with conductive metal, it makes it possible to prevent the high frequency noise from being transmitted from the excimer lamps to the outside of the housing 11. This allows control commands to other control systems installed in the vicinity of the UV irradiation unit 10 from being disturbed by the high frequency noise, thereby preventing defects from occurring in the control commands.

As described above, in the UV irradiators 100A to 100F according to the present embodiment, for excimer lamps serving as the UV light source, it is preferable to use KrCI excimer lamps emitting UV light having a peak wavelength of 222 nm, or KrBr excimer lamps emitting UV light having a peak wavelength of 207 nm.

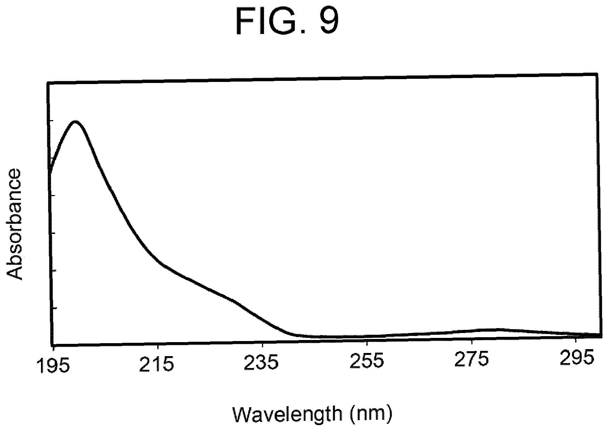

FIG. 9 is a chart exemplarily illustrating the UV absorption spectrum of protein.

As shown in FIG. 9, it can be observed that the protein has a light absorption peak at a wavelength of 200 nm, while UV light having a wavelength of 240 nm or longer is unlikely to be absorbed by the protein. This means that the UV lights having the wavelength of 240 nm or longer is likely to pass through human skins and further penetrate skin inner tissues. As a result, cells inside the human skins are likely to be damaged. On the other hand, the UV light having the wavelength near 200 nm is absorbed by surfaces of the human skins (e.g., stratum corneum) and do not penetrate the skin inner tissues. Therefore, the UV light having the wavelength near 200 nm is safe for the human skins.

On the other hand, the UV light having a wavelength less than 190 nm may produce more ozone (O₃). This is because, when the UV light having the wavelength less than 190 nm is emitted in an atmosphere containing oxygen, oxygen molecules are more photolyzed to produce oxygen atoms, and ozone is produced by the bonding reaction between oxygen molecules and oxygen atoms. For this reason, it is undesirable to emit the UV light having a wavelength less than 190 nm in the atmosphere. Furthermore, it is desirable to use UV light having a peak wavelength of 200 nm or longer to more effectively suppress ozone generation in the atmosphere.

For this reason, the wavelength range between 190 nm and 240 nm is safe for humans and animals. Furthermore, the wavelength range that is safe for humans and animals is preferably 190 nm to 237 nm, more preferably 190 nm to 235 nm, and even more preferably 190 nm to 230 nm. Yet furthermore, from the viewpoint of more effectively suppressing ozone generation, the wavelength range is preferably 200 nm to 237 nm, more preferably 200 nm to 235 nm, and even more preferably 200 nm to 230 nm.

In other words, UV light having the wavelength of 222 nm emitted from KrCI excimer lamps and UV light having the wavelength of 207 nm emitted from KrBr excimer lamps are both safe for humans and animals, and capable of sterilizing microorganisms and inactivating viruses. Therefore, sterilization and inactivation operations using UV irradiation can be performed even when humans or animals are present in the sterilization and inactivation area in the space.

As described above, according to the checkout system of the present embodiment, when an item related to the checkout processing is transferred to the checkout area where the checkout processing for products 400 is performed by a cashier at the cash register counter 200, the checkout system is capable of detecting the item being transferred to the checkout are by a sensor, and irradiating the checkout area with the UV light in the wavelength range between 190 nm and 240 nm, as the UV light having the wavelength that inactivates microorganism and/or virus.

As a result, it makes it possible to decontaminate items related to the checkout processing more appropriately in the checkout area at the cash register counter 200 and prevent harmful microorganisms and/or viruses from infecting humans through the items. In addition, since the UV light in the wavelength range between 190 nm and 240 nm, which have little adverse effects on cells of humans and animals, are irradiated, it makes it possible to perform the inactivation processing by UV irradiation even in a space where a human is present.

More specifically, the checkout system according to the present embodiment includes the UV irradiators that are capable of irradiating the processing area 211, the standby area 212, the reading area 213, the payment area 214, respectively, with the UV light, each of which is included in the checkout area. The checkout system irradiates the above respective areas with UV light when the sensor detects that an item related to the checkout processing is transferred to any of the above areas. Here, the items related to the checkout processing include an unpaid product 400, a shopping basket containing the product 400, and cash or a card for payment for the product 400.

As such, it makes it possible to irradiate the products 400 in the shopping basket in process 401 and checkout-standby shopping basket 402, as well as 400 products during the checkout processing, which are being grasped by the cashier 301 (or customer 302 in the case of a self-checkout system), with the UV light, so as to appropriately inactivate microorganisms and/or viruses adhered to the surface of the products 400. This allows the cashier 301 to perform the checkout processing for the products 400, which has been decontaminated. Also, the customer 302 may take home the products 400 in the decontaminated state. Thus, it makes it possible to prevent harmful microorganisms and/or viruses from infecting the cashier 301 or the customer 302 through the products 400.
Furthermore, the UV light is also appropriately irradiated onto cash, cards, or the like, which are delivered between the cashier 301 and the customer 302, thus, it also makes it possible to prevent infection of harmful microorganisms and/or viruses from person to person.

As described above, the checkout system according to the present embodiment is capable of appropriately decontaminate the products 400 to be checked out, which may be in contact with an unspecified number of people and may have viruses and/or microorganisms adhered thereto. Therefore, it makes it possible to appropriately suppress the secondary infection of viruses and/or microorganisms from occurring on the cashier 301 or the customer 302 when the cashier 301 comes into contact with the products 400 or when the customer 302 takes the products 400 to go.

Furthermore, when the UV light source is installed higher than people's face, such as by mounting the UV light source on the ceiling, as in the UV irradiator 100A, the UV light may be irradiated in the space where people's breath is expelled in the checkout area. Therefore, it makes it possible to inactivate microorganisms and/or viruses attached to droplets in the exhaled breath before the droplets spread.

### Modification to Embodiments

Although the above embodiment describes a certain case in which excimer lamps are used as the UV light source, alternatively, solid-state light sources such as LEDs and LDs may also be used as the UV light source.

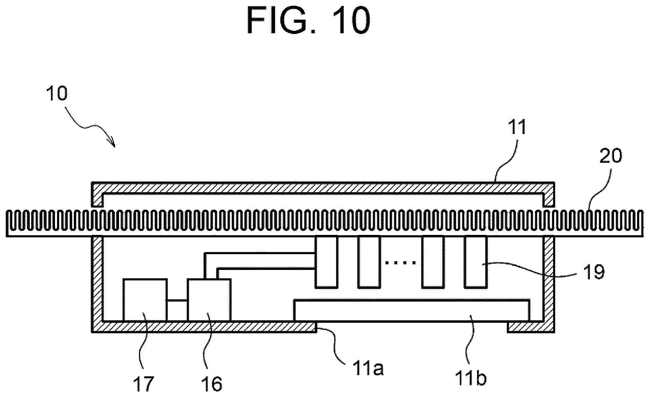

FIG. 10 is a schematic diagram illustrating an exemplary configuration of a UV irradiation unit 10 using an LED 19 as the UV light source. Referring to FIG. 10, the UV irradiation unit 10 includes multiple LEDs 19.

As described above, the wavelength range of UV light used for decontamination (or sterilization) applications is between 200 and 320 nm, and a particularly effective wavelength is around 260 nm, where nucleic acids (i.e., DNA and RNA) are highly absorbed.

Therefore, the LED 19 as the UV light source in the UV irradiation unit 10 is also employed to emit UV light having the wavelength between 200 and 320 nm. More specifically, for example, aluminum gallium nitride (AlGaN)-based LEDs, aluminum nitride (AIN)-based LEDs, and the like, may be employed. The AIGaN-based LEDs emit light in the deep UV (i.e., DUV) wavelength range between 200 and 350 nm by changing the composition of Al. the AIN-based LEDs emit UV light having a peak wavelength of 210 nm.

Here, for AIGaN-based LEDs, it is preferable to adjust the composition of Al such that the center wavelength is in the range between 200 nm and 237 nm. As described above, the UV light within such wavelength range is safe for humans and animals and thus able to appropriately sterilize microorganisms and inactivate viruses. For example, by adjusting the composition of Al, it makes it possible to make an AIGaN-based LED having the center wavelength of 222 nm of the emitted UV light.

Magnesium zinc oxide (MgZnO)-based LEDs may also be employed as LEDs, which emit light in the deep UV (DUV) wavelength range between 190 nm and 380 nm by changing the composition of magnesium (Mg).

Here, for MgZnO-based LEDs, it is preferable to adjust the composition of Mg such that the center wavelength is in the range between 200 nm and 237 nm.
As described above, UV light within such wavelength range is safe for humans and animals and thus able to appropriately sterilize microorganisms and inactivate viruses. For example, by adjusting the composition of Mg, it makes it possible to make an MgZnO-based LED having the center wavelength of 222 nm of the emitted UV light.

Here, LEDs that emit UV light (in particular, UV light in the deep UV region) as described above have a low luminous efficiency of several percent or less and generate a large amount of heat. Also, when the heat generation of LEDs increases, the intensity of light emitted from such LEDs decreases, and a wavelength shift of the emitted light also occurs. Therefore, in order to suppress the heat increase of LEDs, it is preferable to install the LED 19 on a cooling member 20 (e.g., heat dissipating fins that dissipate heat) as shown in FIG. 10.

At this time, as shown in FIG. 10, a part of the cooling member 20 may protrude from the housing 11 of the UV irradiation unit 10.

It should be noted that the AIGaN-based LEDs and MgZnO-based LEDs that emit UV light having the center wavelength of 222 nm described above emit UV light in the wavelength range with some spread from the center wavelength of 222 nm, and the light emitted from such LEDs includes, although only slightly, UV light with wavelengths unsafe for humans and animals. Therefore, as in the case where the UV light source is an excimer lamp, it is preferable to employ a dielectric multilayer filter (i.e., optical filter) that cuts light in the UV-C wavelength range with wavelengths other than the wavelength range between 200 nm and 237 nm.

The optical filter above is preferably one that cuts light in the UV-C wavelength range with wavelengths other than 200 nm to 235 nm, and even more preferably one that cuts light in the UV-C wavelength range with wavelengths other than 200 nm to 230 nm. The same is true when the light source is an excimer lamp.

However, AIN-LEDs emitting UV light having a center wavelength of 210 nm above do not require the above optical filter.

Regardless of the ultraviolet light source being an excimer lamp or an LED, in some cases, the illuminance of UV light of unsafe wavelengths for humans and animals at the UV-irradiated surface may drop down to the allowable value or less, depending on the illuminance at the light emitting surface of the UV light source and the distance from the UV light source to the UV-irradiated surface. Therefore, in such cases, it is not required to employ the optical filter described above.

It should be noted that, although the above embodiment describes a certain case in which the checkout counter is a cash register counter at convenience stores, supermarkets, department stores, or the like, the present embodiment is not limited to the above. For example, the checkout counter may be a lending counter in libraries. In this case, during the checkout processing (e.g., lending processing) by the staff at the lending counter, it makes it possible to irradiate the lending counter with the UV light in the wavelength range between 190 nm and 240 nm to inactivate harmful microorganisms and/or viruses adhered to the surface of books to be lent.

As described above, according to the inactivation method of the present invention, it makes it possible to provide the inherent sterilizing and virus inactivation capabilities of UV light without incurring the adverse effects of UV irradiation on human bodies. In particular, unlike conventional UV light sources, it makes it possible to effectively perform the inactivation processing using UV light even in spaces where a human is present. This addresses Goal 3 of the United Nations-driven Sustainable Development Goals (SDGs): "Ensure healthy lives and promote well-being for all people of all ages," and make a significant contribution to Target 3.3: "By 2030, end the epidemics of AIDS, tuberculosis, malaria and neglected tropical diseases and combat hepatitis, water-borne diseases and other communicable diseases."

### REFERENCE SIGNS LIST

10: UV Irradiation Unit; 11: Housing; 12: Excimer Lamp; 16: Power Supply Unit; 17: Control Unit; 19: LEDs; 100A to 100F: UV Irradiation Apparatuses (UV Irradiators); 200: Cash Register Counter; 201: Partition; 202: Aperture; 203: UV Transmissive Window; 204: Reflective Surface; 211: Processing Area; 212: Standby Area; 213: Reading Area; 214: Payment Area; 22: Cash Register; 221: Reader; 240A to 240B: Product Conveyance Mechanisms; 241: Product Passage Member; 242: Entrance; 243: Exit; 301: Cashier; 302: Customer; 400: Product; 401: Shopping Basket in Process; 402: Checkout-Standby Shopping Basket; 403: Checked-out Shopping Basket; 1000A to 1000C: Checkout Systems

## Claims

1. An inactivation method of emitting ultraviolet light in a space where a human is present to inactivate microorganisms and/or viruses existing in the space, comprising:
a detection step for detecting an item related to checkout processing, the item being transferred to a checkout area where the checkout processing is performed for the item by a cashier at a checkout counter;
an irradiation step for controlling, when the item is detected in the detection step, an ultraviolet light source that emits ultraviolet light in a wavelength range between 190 nm and 240 nm as ultraviolet light having a wavelength that inactivates the microorganisms and/or viruses and irradiating the checkout area with the ultraviolet light; and
a removing step for transferring the item from the checkout area after the item has been irradiated with the ultraviolet light.

2. The inactivation method according to Claim 1, wherein
the irradiation step irradiates the checkout area with the ultraviolet light from a plurality of directions.

3. The inactivation method according to Claim 1, wherein
the detection step detects that an item is placed in a processing area on the checkout counter, the processing area being an area included in the checkout area and in which the item is to be placed during the checkout processing; and
the irradiation step irradiates the processing area with the ultraviolet light when the detection step detects that the item is placed in the processing area.

4. The inactivation method according to Claim 1, wherein
the detection step detects that an item is placed in a standby area on the checkout counter, the standby area being an area included in the checkout area and in which the item awaiting the checkout processing is to be placed; and
the irradiation step irradiates the standby area with the ultraviolet light when the detection step detects that the item is placed in the standby area.

5. The inactivation method according to Claim 1, wherein
the item is a to-be-purchased product and the checkout processing is processing for checking out the to-be-purchased product.

6. The inactivation method according to Claim 5, wherein
the detection step detects that an item is present in a reading area for reading product information assigned to the to-be-purchased product by a reader that reads the product information, the reading area being an area included in the checkout area; and
the irradiation step irradiates the reading area with the ultraviolet light when the detection step detects that the item is present in the reading area.

7. The inactivation method according to Claim 5, wherein
the detection step detects that an item is present in a payment area for paying for the to-be-purchased product, the payment area being an area included in the checkout area; and
the irradiation step irradiates the payment area with the ultraviolet light when the detection step detects that the item is present in the payment area.

8. The inactivation method according to any one of Claims 1 to 7, wherein
the irradiation step stops irradiation of the ultraviolet light triggered by an event that the detection step no longer detects the item.

9. The inactivation method according to any one of Claims 1 to 7, further comprising:
a measurement step for measuring an irradiation amount of the ultraviolet light to the cashier, wherein
the irradiation step stops irradiation of the ultraviolet light when the irradiation amount to the cashier reaches an allowable upper limit of the irradiation amount to the cashier.

10. A checkout system comprising:
a checkout counter;
a checkout apparatus installed in the checkout counter; and
an ultraviolet light irradiation apparatus including an ultraviolet light source configured to emit ultraviolet light in a wavelength range between 190 nm and 240 nm as ultraviolet light having a wavelength that inactivates the microorganisms and/or viruses, and a control unit configured to control irradiation of the ultraviolet light by the ultraviolet light source,
the ultraviolet light irradiation apparatus further including a detection unit configured to detect an item related to checkout processing, the item being transferred to a checkout area where the checkout processing is performed for a to-be-purchased product by a cashier at the checkout counter,
the ultraviolet light source being configured to irradiate the checkout area with the ultraviolet light, and
the control unit being configured to control, when the item is detected by the detection unit, the ultraviolet light source to irradiate the checkout area with the ultraviolet light to irradiate the item with the ultraviolet light.

11. The checkout system according to Claim 10, wherein
the ultraviolet light source is configured to irradiate the checkout area with the ultraviolet light from at least one of above and below the checkout area.

12. The checkout system according to Claim 10, wherein
the ultraviolet light source is configured to irradiate a processing area on the checkout counter with the ultraviolet light, the processing area being an area included in the checkout area and in which the to-be-purchased product is to be placed during the checkout processing,
the detection unit detects that an item is placed in the processing area on the checkout counter, and
the control unit controls the ultraviolet light source to irradiate the processing area with the ultraviolet light when the detection unit detects that the item is placed in the processing area.

13. The checkout system according to Claim 10, wherein
the ultraviolet light source is configured to irradiate a standby area on the checkout counter with the ultraviolet light, the standby area being an area included in the checkout area and in which the to-be-purchased product awaiting the checkout processing is to be placed,
the detection unit detects that an item is placed in the standby area on the checkout counter, and
the control unit controls the ultraviolet light source to irradiate the standby area with the ultraviolet light when the detection unit detects that the item is placed in the standby area.

14. The checkout system according to Claim 10, wherein
the ultraviolet light source is arranged in a reader that reads product information assigned to the to-be-purchased product,
the detection unit detects that an item is present in a reading area for reading the product information by the reader, the reading area being an area included in the checkout area, and
the control unit controls the ultraviolet light source to irradiate the reading area with the ultraviolet light when the detection unit detects that the item is present in the reading area.

15. The checkout system according to Claim 10, wherein
the ultraviolet light source is configured to irradiate a payment area for paying for the to-be-purchased product, the payment area being an area included in the checkout area,
the detection unit detects that an item is present in the payment area, and
the control unit controls the ultraviolet light source to irradiate the payment area with the ultraviolet light when the detection unit detects that the item is present in the payment area.

16. The checkout system according to Claim 15, wherein
the checkout counter is provided with a partition made of transparent material with an aperture for paying for the to-be-purchased product, the partition being arranged between a purchaser of a product and a cashier who performs the checkout processing and is different from the purchaser,
the ultraviolet light source is arranged at the aperture, and
the detection unit detects that the item is present in the payment area that is formed by the aperture.

17. The checkout system according to any one of Claims 10 to 16, wherein
the control unit controls the ultraviolet light source to stop irradiation of the ultraviolet light triggered by an event that the detection unit no longer detects the item.

18. The checkout system according to any one of Claims 10 to 16, further comprising:
a measurement unit configured to measure an irradiation amount of the ultraviolet light to the cashier, wherein
the control unit controls the ultraviolet light source to stop irradiation of the ultraviolet light when the irradiation amount measured by the measurement unit reaches an allowable upper limit of the irradiation amount.

19. The checkout system according to Claim 10, wherein
at least an area including and adjacent to an area in which the to-be-purchased product is to be placed on a surface of the checkout counter constitutes a reflective surface that reflects the ultraviolet light.

20. The checkout system according to Claim 10, wherein
the checkout counter is provided with a window that transmits the ultraviolet light on a surface of the checkout counter, at least in an area in which the to-be-purchased product is to be placed, and
the ultraviolet light source is arranged inside the checkout counter and below the window and configured to emit the ultraviolet light in an upward direction.

21. The checkout system according to Claim 10, wherein
the checkout counter is provided with a product conveyance mechanism that loads and conveys the to-be-purchased product to a checkout position where the checkout processing is performed by the cashier, and
the ultraviolet light source is configured to irradiate the ultraviolet light onto an area upstream in a conveyance direction of the to-be-purchased product from the checkout position on a conveyance path of the to-be-purchased product by the product conveyance mechanism.

22. The checkout system according to Claim 21, wherein
the product conveyance mechanism is provided with a belt with a predetermined gap or rollers that rotate at a predetermined distance apart as a conveyance path on which the to-be-purchased product is to be placed, and
the ultraviolet light source is arranged below the conveyance path and emits the ultraviolet light in an upward direction.

23. The checkout system according to Claim 21, wherein
the conveyance path is constituted with rollers that rotate at a predetermined distance apart, surfaces of the rollers constitute reflective surfaces that reflect the ultraviolet light, and
the ultraviolet light source is configured to irradiate the rollers of which surfaces constitute the reflective surfaces with the ultraviolet light.

24. The checkout system according to Claim 21, wherein
the checkout counter is provided with a product passage member with an entrance/exit through which the to-be-purchased product conveyed by the product conveyance mechanism may pass, upstream in a conveyance direction of the to-be-purchased product from the checkout position on the conveyance path, and
the ultraviolet light source is arranged inside the product passage member.

25. The checkout system according to Claim 24, wherein
at least a part of an inner surface of the product passage member constitutes a reflective surface that reflects the ultraviolet light.
